# EUROPEAN PATENT APPLICATION

(11) **EP 4 592 395 A1**
(43) Date of publication of application: **30.07.2025**
(21) Application number: 24154183.8
(22) Date of filing: 26.01.2024
(51) Int. Cl.: C12P 5/02, B65D 65/46, C12P 7/42, C12P 7/52, C12P 7/54, C12P 7/625

(54) **A METHOD FOR PRODUCING POLYHYDROXYALKANOATES**

(71) Applicant: Fortum Waste Solutions Oy, 11120 Riihimäki (FI)
(72) Inventor: WILLQUIST, Karin, 02150 Espoo (FI); SUDHANSHU, Pawar, 02150 Espoo (FI); TOROPAINEN, Jarkko, 02150 Espoo (FI); KOIVUNIEMI, Mikko, 02150 Espoo (FI)
(74) Representative: Laine IP Oy

(57) **Abstract**

According to an example aspect of the present invention, there is provided A method for producing polyhydroxyalkanoates. The method comprises the steps of: converting carbon dioxide to one or more organic compound(s), contacting at least part of the one or more organic compound(s) with a microbial monoculture and a fermentation medium to form a fermentation mixture, and producing at least one polyhydroxyalkanoate from the fermentation mixture.

## Description

### FIELD

The invention belongs to the field of polymer production. More specifically the invention relates to production of polymers from waste materials and products obtainable with such methods.

### BACKGROUND

The need for plastics is expected to increase to 1100 Mt by 2050 and is expected to contribute to 19% of the world's greenhouse gas emission. Even when 63% of this plastic is expected to be recycled, there are limitations in current recycling method resulting in residual plastics waste that is either too complex or too hazardous to be recycled with current methodology. Today 90% of the plastics is produced from fossil sources. Only 9% is recycled due to the challenges with chemical (pyrolysis) and mechanical recycling (ref UNEP). Thus, the waste is mostly landfilled while part is incinerated with energy recovery.

Biodegradable polymers such as polyhydroxyalkanoates (PHA) can be produced by microbial fermentation. For example, polyhydroxybutyrate (PHB) can be produced from methane using preferable mixed cultures with cell debris recirculation (WO2011031566). However, such PHB is known to be brittle, limiting its usability. To increase elasticity, poly(3-hydroxybutyrate-co-3-hydroxyvalerate) (PHBV) can be produced from methane by the addition of propionate or valerate in the fermentation medium. However, the cost of valerate can reach 50 euro/kg, which has a great influence on the production cost of PHBV. In addition, while PHAs can be produced from various substrates, they are mostly produced from starch or lipids. Thus, the production of PHAs typically competes for the same raw material as food production, which has ethical challenges.

One possible raw material utilized for PHA production, especially for production of PHBV, is volatile fatty acids (VFAs) derived from biogas from municipal biological waste or biosludge from municipal water treatment. Such process has been described for example in EP3148942. However, it is difficult to get sufficiently high quality VFA to support an economically feasible PHBV production. Another challenge is the mixed culture used by the waste treatment plants, which makes further processing of biosludge challenging. To get a very high and consistent PHBV quality it would be greatly beneficial to have a genetically modified bacterial strain that can display high yield and quality. However, to maintain such culture in dominance it is necessary to have a sterile operation. This is not possible when using typical VFA origins, such biosludge.

### SUMMARY OF THE INVENTION

The invention is defined by the features of the independent claims. Some specific embodiments are defined in the dependent claims.

According to a first aspect of the present invention, there is provided a method for producing polyhydroxyalkanoates. The method comprises the steps of: converting carbon dioxide to one or more organic compound(s), contacting at least part of the one or more organic compound(s) with a microbial monoculture and a fermentation medium to form a fermentation mixture, and producing at least one polyhydroxyalkanoate from the fermentation mixture.

According to a second aspect of the present invention, there is provided a product obtainable with the method according to the first aspect of the invention.

According to a third aspect of the present invention, there is provided a use of the product according to the second aspect of the invention as a packaging material.

### Advantages of the invention

An advantage of the present invention is that the invention may allow turning materials typically considered as waste into bioplastic materials.

A further advantage of the present invention is that it may allow the use of microbial monoculture in the production of the bioplastic material.

Another advantage of the present invention is that it may allow circulating the formed microbial cell debris back to the fermentation stage. Such process allows decreasing the overall amount of produced waste.

A further advantage of the present invention is that efficient production of bioplastics may be achieved even without addition of purified volatile fatty acids, nitrogen or nutrients.

Another aspect of the present invention is, that the method allows transforming mixed and/or hazardous waste materials to bioplastic.

Yet another advantage of the invention is that it allows minimizing the amount of waste produced during the production of bioplastic.

### BRIEF DESCRIPTION OF THE DRAWINGS

Figure 1 illustrates a process line capable of supporting at least some embodiments of the present invention.
Figure 2 illustrates another process line capable of supporting at least some embodiments of the present invention.

### EMBODIMENTS

### DEFINITIONS

In the present context, the terms "polyhydroxyalkanoates" or "PHA" refer to a group of polyesters produced in the nature by numerous microbes. Typically, they are fermented from sugars or lipids. More than 150 different monomers can be combined within this family to give materials with extremely different properties. The formed polyesters are biodegradable and can be used in the production of bioplastics.

In the present context, the terms "hydrothermal carbonization" or "HTC", also sometimes referred to as aqueous carbonization at elevated temperature and pressure, refer to a thermochemical process in which biomass is decomposed under heat and pressure. Hydrothermal carbonization produces a liquid fraction, a gaseous product and hydrochar. The conditions during hydrothermal carbonization can vary. In some examples, HTC takes place at temperatures ranging from 150 to 300°C. The liquid fraction comprises different decomposition products in an aqueous medium. The exact composition of the liquid fraction depends on the used biomass and parameters during the treatment, but typically it may comprise reducing sugars, furan compounds, such as furfural, furfuryl alcohol, hydroxymethylfurfural, volatile fatty acid, such as succinic acid, lactic acid, formic acid, acetic acid, levulinic acid, and propionic acid, and phenolic compounds.

In the present context, the term "slow pyrolysis" refers to slow heating of organic material in absence of oxygen. Instead of combusting, the volatiles from the organic material evaporate partly. Slow pyrolysis typically runs at temperatures in the range 300 to 700 °C, in some instances in the range 400 to 500 °C, and has slow heating rates and longer vapour residence times. Such process produces a fluid fraction. Typically, the vapour residence time in slow pyrolysis ranges from 10 to 100 minutes. Slow pyrolysis is more tolerant to moisture content in feedstock than fast pyrolysis.

In the present context, the term "hydrothermal liquefaction" refers to a thermal depolymerization process used to convert wet biomass into a fluid under moderate temperature and high pressure. The process has also been called hydrous pyrolysis. Most applications of hydrothermal liquefaction operate at temperatures between 250-550 °C and high pressures of 5-25 MPa, although higher or lower temperatures can be used to optimize gas or liquid yields, respectively. In some examples a catalyst may be used. At these temperatures and pressures, the water present in the biomass becomes either subcritical or supercritical, depending on the conditions, and acts as a solvent, reactant, and catalyst to facilitate the reaction of biomass to a fluid. The exact composition of the liquid fraction depends on the used biomass and parameters during the treatment.

In the present context, the term "hydrochar" refers in present disclosure to a solid product obtained by hydrothermal carbonization of biomass. Hydrochar is formed primarily from the thermochemical conversion of lignocellulosic material, in particular, lignin. In addition, hydrochar can be derived from carbon capture technologies. Hydrochar, is composed mainly of carbon, preferably comprising at least 20 wt-%, such as 20 to 99.9 wt-% carbon.

In the present context the term "fermentation" refers to a process of applying microbes for the production of compounds. In a typical process, a liquid medium is inoculated with a microbial culture and the microbes are grown under specific conditions. At a suitable timepoint, the production of the desired chemical may be induced. Such induction is typically done by addition of a chemical, and/or by changing conditions, such as temperature and/or pressure. The liquid medium comprises nutrients that allow the microbial culture to grow and may be optimized for the used microbial culture. The microbial culture typically comprises bacterial and/or yeast cells in a monoculture or in a polyculture. A monoculture consists of a single microbial species whereas a polyculture may comprise multiple different microbial species. A fermentation process may be aerobic or anaerobic.

In the present context, the term "culture" refers to a population of unicellular or multicellular microorganisms in a medium, such as a growth or fermentation medium.

In the present context, the terms "volatile fatty acids" or "VFAs" refer to short chain fatty acids that are water soluble and may be separated by steam distillation at atmospheric pressure. Volatile fatty acids typically comprise carbon backbones of 1 to 6 carbon atoms. Examples of volatile fatty acids include but are not limited to acetic acid, propionic acid, butyric acid, valeric acid, isovaleric acid and succinic acid. In the present context, the term "volatile fatty acids" refers also to salts or esters of volatile fatty acids. Examples of salts of volatile fatty acids include acetate, propionate, butyrate, valerate, isovalerate and succinate.

The present disclosure provides a process for the production of polyhydroxyalkanoates (PHA) from waste materials. In more detail, the process comprises the steps of converting the carbon dioxide to one or more organic compound(s), contacting at least part of the one or more organic compound(s) with a microbial monoculture and with a fermentation medium to form a fermentation mixture, and producing at least one polyhydroxyalkanoate from the fermentation mixture.

In some embodiments, the carbon dioxide may be obtained from incineration or combustion of waste material or from industrial processes, such as pulp and paper industry. Any waste materials may be used. Especially suitable waste materials include those that are difficult to recycle or use by other means. Such waste materials may comprise for example mixed waste, such as municipal waste, hazardous waste, biomass, wastewater, condensate streams, or the like. In some embodiments, the waste material comprises mixed waste or hazardous waste. In some cases, the waste material may comprise fossil fuels and/or biofuels, such as at least one fuel or mixed fuels, including support fuels, such as waste solvents and waste oils. A fuel may be selected from diesel, gasoline, kerosene, ethanol, acetone, ethyl acetate, toluene, coal, propane, methane or hydrogen. Carbon dioxide may also be obtained from different industrial processes that produce carbon dioxide as a side product. Such industries include for example the pulp and paper production, energy production or production of building materials.

In the disclosed process, carbon dioxide is first converted to one or more organic compounds. Such conversion can be achieved by any known methods, such as for example by microbial fermentation, electrochemical methods or by hydrogenation. As will be understood by the skilled person, the process can be direct hydrogenation of carbon dioxide into the organic compound or the hydrogenation can proceed through a multistep reaction to obtain the organic compound. Preferably, the converting of carbon dioxide to one or more organic compound(s) comprises or consists of microbial fermentation, such as bacterial fermentation. Microbes capable of converting carbon dioxide to one or more organic compounds are known in the art and a person skilled in the art is well-equipped to choose appropriate conditions for such fermentation process. In some embodiments, carbon dioxide may be converted to C1-compounds. The term "C1-compound" refers in the present disclosure to a compound, which contains only one carbon atom. Examples of C1-compounds include but are not limited to formate, formic acid, formamide, formaldehyde, carbon monoxide, methane, methanol, methylamine, halogenated methanes, and monomethyl sulfate. Carbon dioxide may in other examples be converted to C2-compounds. The term "C2-compounds" refers in the present disclosure to a compound which contains two carbon atoms. Examples of C2-compounds include but are not limited to ethanol, acetate and acetic acid. Carbon dioxide may also in some embodiments be converted to organic acids, such as volatile fatty acids. In some embodiments, the organic compound(s) are selected from methane, methanol, acetate, butyrate, valerate, or combinations thereof. Preferably, the organic compound may be methane.

The formed organic compounds are then contacted with a microbial monoculture. A microbial monoculture comprises only a single type of microbe. In some embodiments, the microbial monoculture consists of one microbial species, preferably one bacterial species. The advantage of a monoculture is that the fermentation process of a monoculture is easier to control and provides typically more consistent product than a polyculture, which comprises at least two but typically several different microbes. This is the result of having just one microbial species in the fermentation medium. A monoculture is possible to achieve only in a sterile environment and thus the use of monocultures is difficult to realize in many process setups. Simple exchanging a polyculture in one process line with a monoculture is not typically possible because means for sterile fermentation are not present is a process line that has been designed for polycultures.

Preferably, the microbial culture is a bacterial culture. The microbial culture must be able to produce polyhydroxyalkanoates. A suitable microbial culture may comprise for example a methylotrophic microbe. Methylotrophic microbes are microorganism that are able to use C1-compounds as an energy or carbon source for their growth and development. Methylotrophic microbes often use C1-compounds as a source of energy and as a source of carbon. Selecting certain types of organic compound(s) may have advantages in the further processing steps. For example, methanol may limit growth of many microbes and thus it can be used to minimize contaminations in the further fermentation steps. Non-limiting examples of suitable microbial species include for example *Cupriavidus necator, Methylorubrum extorquens, Paracoccus denitrificans* and *Methylobacterium organophilum.*

In some embodiments, the fermentation medium may comprise volatile fatty acids. Volatile fatty acids may comprise for example acetic acid, propionic acid, butyric acid and/or valeric acid. In some examples, volatile fatty acids may be provided in the form of a salt. Non-limiting examples of volatile fatty acid salts include acetate, propionate, butyrate, and/or valerate. It has been observed, that when PHAs are fermented from only C1-carbon sources, such as carbon dioxide, methanol or methane, the fermentation yields simple structures, such as polyhydroxybutyrate (PHB). PHB is brittle and can thus can be used only in limited applications. An advantage of using volatile fatty acids in the fermentation medium is that more complex PHA structures, such as poly(3-hydroxybutyrate-co-3-hydroxyvalerate, PHBV), may be obtained from fermentation medium comprising volatile fatty acids. Compounds comprising three or five carbons (C3 or C5 compounds) have been observed to be especially well-suited for formation of more complex PHA structures, such as PHBV. In some preferred embodiments, the volatile fatty acid is selected from propionic acid and/or valeric acid. In some embodiments, instead of volatile fatty acids other fatty acids that can be converted to volatile fatty acids during the fermentation step may be used.

In some embodiments, the fermentation medium may comprise a fluid obtained from decomposition of a biomass. The term "fluid obtained from decomposition of biomass" may refer to a water-soluble liquid product and may be obtainable from (thermo)chemical decomposition of biomass. One advantage observed with using a fluid obtained from decomposition of a biomass is that such fluid is rich for example in nutrients, nitrogen and carbon sources. Thus, use of a fluid obtained from decomposition of a biomass in fermentation medium may reduce the cost associated with production of fermentation medium. The fluid obtained from decomposition of a biomass may comprise lipids and/or a nitrogen source. In addition, the fluid obtained from decomposition of a biomass may comprise wide range of different nutrients, such as micronutrients including trace elements. The nitrogen in fluid from decomposition of a biomass is typically in the form of ammonium ions, which may perform as nitrogen source for microbes during for fermentation. In some embodiments, the fluid obtained from decomposition of a biomass comprises lipids, such as fatty acids or volatile fatty acids, and/or nitrogen. Thus, in some examples, nitrogen and/or volatile fatty acids do not need to be separately added to the fermentation medium by other means.

In some embodiments, the fluid obtained from decomposition of a biomass may be produced by hydrothermal carbonization, slow pyrolysis, hydrothermal liquefaction, biological treatment, or combinations thereof. These processes produce a fluid fraction which comprises variety of compounds from biological sources. Such processes typically produce in addition to the fluidic fraction a hydrochar or a biochar, which can be used for example as fuel, fertilizers or in water treatment. The term "fluid obtained from decomposition of a biomass" does not refer to a hydrochar or biochar produced in these processes.

The term "biological treatment" in the present context refers to treatment of biomass with microbes, such as bacteria and/or fungi, and/or enzymes to degrade the biomass into a fluid fraction. Typically, a mixture of different microbes and/or enzymes is utilized in a biological treatment. In some examples, biological treatment may refer to enzymatic degradation of biomass. In other examples, biological treatment may refer to microbial degradation of biomass. In some examples, the biological treatment comprises acidification of the biomass.

The term "biomass" is utilized in the present disclosure with regard to any material that has biological origin. Biomass may be plant-based material, typically available on renewable basis, including products of agriculture and forestry, as well as residues and waste from wood-, paper and food industries. Biomass, may also be microbial-based, for example cell debris from a fermentation process. Biomass may in some examples be derived from animal sources, such as waste from food industry. Biomass may also be combination of biomass from different origins, which is advantageous because for example waste from food industry or municipal biowaste does not have to be separated prior its use in the decomposition process.

In some embodiments, biomass may comprise or consist of biosludge, such as biosludge from a water treatment plant. It is advantageous that the biomass comprises waste or side-stream material in order to promote circular economy. In some preferred embodiments, decomposition of biomass takes place at high temperatures, such as ranging from 180 to 700 °C, for extended period of time, typically in the range of several hours, and in some occasions even under elevated pressure. Thus, the liquid derived from such process is sterile. Sterility is a requirement so that the liquid may be used in a fermentation process that utilized a monoculture of microbes. Thus, the process producing fluid from decomposition of a biomass may have conditions that allow production of a sterile fluid. For example, the temperature during production of fluid from decomposition of a biomass may be at least 121 °C. The fluid obtained from decomposition of a biomass may also be sterilized after the fluid has been produced. However, obtaining a sterile fluid during decomposition is advantageous as it reduces the complexity of the overall process. The exact temperature for achieving sterility of a fluid depends on used time and pressure. A person-skilled in the art is able to determine conditions in which a fluid obtained from decomposition of biomass is sterile.

The fermentation process is optimized for the used microbial cultures. Typically, the fermentation takes place in a fermentation reactor in which the conditions may be optimized for the production of the desired product(s). A fermentation reactor comprises a fermentation medium into which the microbial culture, and organic compound(s) are added to. The fermentation medium typically comprises vitamins and minerals that are needed for optimal microbial growth and for the production of the PHAs. In addition, the fermentation medium must comprise a carbon source and a nitrogen source. In some embodiments of the present disclosure, these can be provided by adding a fluid obtained from decomposition of a biomass to the fermentation medium as explained above. Temperature during the fermentation process may be optimized for the used microbial culture, but is typically in the range of 20 to 60 °C. Typically, fermentation is performed under atmospheric pressure. In some occasions, CO₂ may be utilized as a carbon source for the microbial culture and in such cases pressure up to 10 bar may be utilized in the fermentation reactor.

The fermentation process may be operated as a continuous, batch or fed-batch process. The fermentation reactor and the fermentation medium are first sterilized and subsequently inoculated with a culture of the desired microorganism in the presence of all the required nutrients including oxygen and the carbon source. An oxygen source or air may be continuously introduced. In the continuous and fed-batch processes, the organic compounds and/or the liquid from decomposition of biomass are continuously introduced to the fermentation reactor at a rate, which is either predetermined or in response to need. The need may be determined by monitoring parameters, such as concentration of organic compounds, alcohol, dissolved oxygen, and/or oxygen or carbon dioxide in the gaseous effluent from the fermentation reactor.

The fermentation process produces PHAs. In some embodiments, the polyhydroxyalkanoates are formed from the polymerization of 3-hydroxyvalerate, 3-hydroxybutyrate, 3-hydroxyhexanoate, 3-hydroxypropionate, 3-hydroxyoctanoate or combinations thereof. In some preferred embodiments, the produced PHAs comprise or consist of poly(3-hydroxybutyrate-co-3-hydroxyvalerate), poly-3-hydroxyvalerate or combinations thereof. PHAs may comprise any combination of the above-mentioned monomers. PHAs may comprise the monomers in any ratio. In some embodiments, the ratio between hydroxyvalerate and hydroxybutyrate in the formed PHAs ranges from 1:10 to 10:1, for example from 1:5 to 5:1. In some preferred embodiments, 5 to 50 % of the monomers in the formed PHAs are hydroxyvalerate monomers, such as 3-hydroxyvalerate monomers. For example, the formed PHA may comprise 3-hydroxyvalerate in the range of 10 to 40wt%, or in the range of 15 to 30w% calculated from the total weigh of the polyhydroxyalkanoate.

After the PHAs have been formed, they may be isolated from the fermentation broth by extraction. Thus, the process may comprise an extraction step after the fermentation step, said extraction step producing a fraction comprising at least one polyhydroxyalkanoate, and a cell debris fraction. Any suitable method for extracting PHAs from cell debris may be utilized.

The produced cell debris fraction may in some preferred embodiments be subjected to a decomposition process, such as hydrothermal carbonization, slow pyrolysis, hydrothermal liquefaction, or biological treatment, in order to produce a fluid obtained from decomposition of the cell debris fraction. In some embodiments, at least part of the fluid obtained in the decomposition of the cell debris fraction may be used as fermentation medium or as a part thereof. Preferably, the fermentation medium may comprise a fluid obtained from decomposition of the cell debris fraction. In this way the produced cell debris may be utilized in the fermentation process and the waste produced during the method may be minimized.

Subjecting the cell debris fraction to a decomposition process prior to directing the produced fluid to the fermentation process sterilizes the biomass and thus allows the use of monocultures during fermentation. In some preferred embodiments, the cell debris fraction obtained by extraction may be mixed with another biomass, such as biosludge, before subjecting it to a decomposition process. In some embodiments, the cell debris fraction is dewatered or the water content of the cell debris fraction is reduced before subjecting it to a decomposition process.

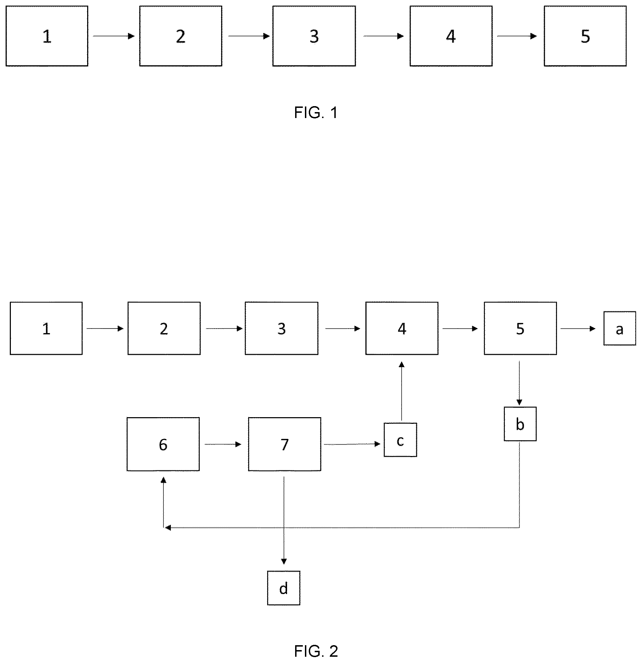

Figure 1 illustrates one embodiment of the method according to the present disclosure. In this example, CO₂ is first produced by waste incineration and collected (1). CO₂ is then converted to at least one organic compound, such as methanol, methane or acetone (2). The conversion may be a microbial process. Next the organic compound(s) are transferred to a fermentation vessel together with a bacterial monoculture (3). The fermentation vessel may in some embodiments comprise also volatile fatty acids so that more complex PHAs may be produced. Next PHAs are accumulated in the fermentation vessel (4). In some embodiments, the PHAs are finally separated from the cell debris formed during fermentation by extraction (5). Such extraction methods are known in the art.

Figure 2 illustrates another embodiment of the present disclosure. This embodiment comprises the same steps 1 to 5 as the embodiment disclosed in figure 1. Shortly, CO₂ is first produced by waste incineration and collected (1), CO₂ is then converted to at least one organic compound (2) and the organic compound(s) are transferred to a fermentation vessel (3) together with a bacterial monoculture. Next PHAs are accumulated in the fermentation vessel (4) and finally, the PHAs (a) are separated from the cell debris (b) by extraction (5). In this embodiment, additionally a biomass is provided (6). Such biomass may be for example biosludge from a water treatment plant but any kind of biomass may be utilized. The biomass is subjected to decomposition process (7) that produced a fluid fraction comprising volatile fatty acids and nitrogen (c). An example of a suitable decomposition process is hydrothermal carbonization process from which the fluid fraction is collected. Such processes typically produce in addition to the fluid fraction a hydrochar or a biochar (d), which can be used for example as fuel, fertilizers or in water treatment. The fluid obtained from decomposition of biomass (c) is transferred to the same fermentation vessel that comprises the organic compound(s) and bacterial monoculture (3). In some additional embodiments, the cell debris (b) may be used as a biomass in step 6. Cell debris may be used as such or mixed with other biomasses, such as with biosludge.

According to another aspect of the present disclosure, there is provided a product obtainable with the above-described method. In some embodiments, the product may comprise at least 50 wt-% of at least one polyhydroxyalkanoate calculated from the dry weight of the product before any extraction of the fermentation product has been done. In preferred embodiments, the product comprises from 50 to 90 wt%, more preferably from 60 to 80 wt-% of at least one polyhydroxyalkanoate calculated from the dry weight of the product before extraction. In some preferred embodiments, the product comprises or consist of poly(3-hydroxybutyrate-co-3-hydroxyvalerate), poly-3-hydroxyvalerate or combinations thereof.

In some embodiments, the product comprises PHBV in which the ratio of 3-hydroxybutyrate and 3-hydroxyvalerate varies between 10:1 and 1:10, for example from 5:1 and 1:5. In some exampled the PHBV comprises at least 10 % of 3-hydroxyvalerate, such as at least 15 %, or at least 20 %, calculated from the total weight of the PHBV. For example, the PHBV may comprise 3-hydroxyvalerate in the range of 5 to 50 %, such as between 10 to 40 % or 15 to 30 %, of the total weight of the formed PHBV.

According to another aspect of the present disclosure, use of the product above disclosed product in or as a packaging material is disclosed.

### Examples

### Example 1

Experiments were performed using methanol as a feedstock for synthesis of PHB using *Methylobacterium extorquens* DSM 1338. The aim of the experiment was fed-batch growth of *Methylobacterium extorquens* using methanol and AIR followed by production of PHA using methanol and AIR.

*Methylobacterium extorquens* DSM 1338 was gown in a fed-batch process at ambient pressure and aerobic conditions. The fermenter was equipped with a pH sensor, a methanol sensor, means for measuring OD (optical density), dissolved oxygen sensor and CO₂/O₂ off gas sensor. 5M NH₄OH was used for controlling pH during the growth phase and mixture of 0.4M NaOH/5M KOH during PHA production phase. Methanol (>99.9% pure) and AMS solution (composition given below) were fed from different feed bottles during the growth phase as well as PHA production phase.

The used culture medium (inoculum and culture media) was identical to the one used by Bourque et al. (1995). It was composed of an autoclavable medium and a filter-sterilised trace element solution. The composition of the autoclavable medium was 1.31 g/L KH₂PO₄, 2.67 g/L Na₂HPO₄·2H₂O, 1.50 g/L (NH₄)₂SO₄, and 0.45 g/L MgSO₄·7H₂O. The trace element solution was concentrated 100 times. The solution was composed of 2.00 g/L FeSO₄ ·7H₂O, 2.00 g/L CaCl₂·2H₂O, 0.49 g/L MnSO₄·H₂O, 0.26 g/L ZnSO₄·7H₂O, 0.08 g/L Na₂MoO₄·2H₂O, 0.08 g/L CuSO₄ ·5H₂O, 0.08 g/L CoCl₂ ·6H₂O, and 0.06 g/L H₃BO₃. In the fed-batch phase, an acidic mineral solution (AMS) was used. The composition of AMS was 270 g/LH₃PO₄, 20 g/L H₂SO₄, 100 g/L MgSO₄·7 H₂O, 2 g/L FeSO₄·7 H₂O, 2 g/L CaCl₂. 2 H₂O, 0.5 g/L MnSO₄. H₂O, 0.08 g/L CoCl2·6 H₂O, 0.26 g/L ZnSO₄·7 H₂O, 0.08 g/L CuCl₂·2 H₂O, 0.08 g/L Na₂MoO₄ · 2H₂O, and 0.06 g/L H₃BO₃.

The results of the fermentation are summarized in table 1:

**Table 1.**

| | |
|---|---|
| **Total C-source consumed** (grams) | 310.00 |
| **Qp** (total PHB produced in grams) | 18.31 |
| **Qrcc** (total biomass produced excluding PHB,grams) | 42.82 |
| **PHB/cell** (PHB content in cells) | 29.9% |
| **µₘₐₓ** (maximum growth rate h⁻¹) | 0.21 |
| **rPHB** (rate of PHB synthesis g/L/h) | 0.10 |
| **HV mol% max** (maximum HV content in PHB) | 0* |

| | |
|---|---|
| *no hydroxyvalerate (HV) was detected in any of the analysed samples. | |

PHB content in the biomass reached 29.9%, defined as ratio of generated PHB to generated biomass at the optimum. The formed PHB polymer did not comprise any hydroxyvalerate (HV) but only 3-hydroxybutyrate.

### Example 2

Experiments were performed with methanol and volatile fatty acids (VFA) as feedstock for PHBV synthesis using *Methylobacterium extorquens* DSM 1338.The experiment aimed at fed-batch growth of *Methylobacterium extorquens* using methanol and AIR followed by production of PHA using methanol, VFAs (VFA_F) and AIR.

*Methylobacterium extorquens* DSM 1338 was gown in a fed-batch process at ambient pressure and aerobic conditions. The fermenter was equipped with a pH sensor, a methanol sensor, means for measuring OD (optical density), dissolved oxygen sensor and CO₂/O₂ off gas sensor. 5M NH₄OH was used for maintaining pH at 6.9 during the growth phase and mixture of 0.4M NaOH/5M KOH during PHA production phase. Methanol (>99.9% pure during growth phase) and AMS solution (composition given below) were fed from different feed bottles. During the PHB production phase, methanol was complemented with acetic acid, propionic acid and butyric acid in following composition (basis of calculation: volatile fatty acids present in 20% mole C compared to methanol) (table 2):

**Table 2.**

| **Compound** | **Acetic acid** | **Butyric acid** | **Valeric acid** | **Methanol** |
|---|---|---|---|---|
| | | | | |
| Added mass (g) | 92.4 | 135.6 | 157.2 | 1000.0 |
| VFA_F mass ratio (g) | 50 | 20 | 30 | |

The used VFA composition resembles the VFA composition in the liquid fraction obtained after the hydrothermal carbonization of organic waste material (Samori, Chiara, et al. 2019).

The used culture medium (inoculum and culture media) was identical to the one used by Bourque et al. (1995). It was composed of an autoclavable medium and a filter-sterilised trace element solution. The composition of the autoclavable medium was 1.31 g/L KH₂PO₄, 2.67 g/L Na₂HPO₄·2H₂O, 1.50 g/L (NH₄)₂SO₄, and 0.45 g/L MgSO₄·7H₂O. The trace element solution was concentrated 100 times. The solution was composed of 2.00 g/L FeSO₄ ·7H₂O, 2.00 g/L CaCl₂·2H₂O, 0.49 g/L MnSO₄ ·H₂O, 0.26 g/L ZnSO₄ ·7H₂O, 0.08 g/L Na₂MoO₄·2H₂O, 0.08 g/L CuSO₄ ·5H₂O, 0.08 g/L CoCl₂ · 6H₂O, and 0.06 g/L H₃BO₃. In the fed-batch phase, an acidic mineral solution (AMS) was used. The composition of AMS was 270 g/LH₃PO₄, 20 g/LH₂SO₄, 100 g/L MgSO₄·7 H₂O, 2 g/L FeSO₄·7 H₂O, 2 g/L CaCl₂. 2 H₂O, 0.5 g/L MnSO₄. H₂O, 0.08 g/L CoCl2·6 H₂O, 0.26 g/L ZnSO₄·7 H₂O, 0.08 g/L CuCl₂·2 H₂O, 0.08 g/L Na₂MoO₄ · 2H₂O, and 0.06 g/L H₃BO₃.

Cultivation was performed in 3 different phases. First the cells were allowed to row by providing sufficient nutrients, carbon source and O₂. In the next phase, the growth was further encouraged by feeding more nutrients and carbon source via fed-batch mode. Lastly, upon reaching the stationary phase, the culture was only fed with carbon-source to allow accumulation of polymer.

Samples from the fermentation medium were taken at specific time points and residual cell concentration (RCC, g.kg⁻¹) and the amount of produced polyhydroxybutyrate (PHB) and polyhydroxyvalerate (PHV) were determined, along with their respective yields (expressed in g generated per g methanol consumed). The results of the fermentation are summarized in table 3:

**Table 3.**

| | |
|---|---|
| **Total C-source consumed** (grams) | 763.00 |
| **Qp** (total PHBV produced in grams) | 21.65 |
| **Qrcc** (total biomass produced excluding PHBV, grams) | 80.21 |
| **PHBV/cell** (PHBV content in cells) | 17.1% |
| **µₘₐₓ** (maximum growth rate h⁻¹) | 0.2 |
| **rPHBV** (rate of PHBV synthesis g/L/h) | 0.10 |
| **HV mol% max** (maximum HV content in PHBV co-polymer) | 19% |

| | |
|---|---|
| HV = hydroxyvalerate | |

PHBV content in the biomass reached 16.6%, defined as ratio of generated PHB to generated biomass, at the optimum time point after which the performance fermentation seemed to decrease as indicated by the amount of produced PHAs. The formed PHBV comprised up to 22% of PHV. At the optimum, PHBV comprised 17% of PHV

### PHB and PHV quantification

PHB(V) quantification analysis was performed by HPLC. Firstly, 10 mg of the lyophilised cell pellets were added to 2 mL of hydrolysis solution (containing 4 g of NaOH, 50 mL of water, and 50 mL of MeOH) and heated to 80 _{°}C for 1 h at 150 rpm. Afterwards, 36 mL of H3PO4 0.05 vol% and 2 mL of HCl 30 vol% were added.
Determination of PHB(V) was carried out with a Prevail^{™} Organic Acid column (5.3 cm × 7 mm). 0.05 % H₃PO₄ and 15 % acetonitrile were used as the eluent at 1 mL/min. The detection was conducted at 215 nm by UV detector (Agilent, USA). The retention phase was 3.5 and 7.3 min for PHB and PHV analysis, respectively. Chromeleon^{™} 7.2 software was used to determine the content of the PHB(V). The PHBV standard (Merck KGaA, Germany) composed of 88 mol% PHB and 12 mol% PHV. PHB(V) was used.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

### ACRONYMS LIST

- AMS: acid mineral solution
- HTC: hydrothermal carbonization
- HV: hydroxyvalerate
- OD: Optical density
- PHA: polyhydroxyalkanoate
- PHB: polyhydroxybutyrate
- PHBV: poly(3 -hydroxybutyrate-co-3 -hydroxyvalerate)
- RCC: Residual cell concentration

### CITATION LIST

Patent Literature
WO2011031566
EP3148942

### Non Patent Literature

Samori, Chiara, et al. "Polyhydroxyalkanoates and crotonic acid from anaerobically digested sewage sludge." ACS Sustainable Chemistry & Engineering 7.12 (2019): 10266-10273.

Bourque, D., Pomerleau, Y. & Groleau, D. High-cell-density production of poly-β-hydroxybutyrate (PHB) from methanol by Methylobacterium extorquens: production of high-molecular-mass PHB. Appl Microbiol Biotechnol 44, 367-376 (1995).

## Claims

1. A method for producing polyhydroxyalkanoates comprising the steps of:
- converting carbon dioxide to one or more organic compound(s),
- contacting at least part of the one or more organic compound(s) with a microbial monoculture and a fermentation medium to form a fermentation mixture, and
- producing at least one polyhydroxyalkanoate from the fermentation mixture.

2. The method according to claim 1, wherein the carbon dioxide is obtained from incineration of waste material or from industrial processes, such as pulp and paper industry.

3. The method according to claim 1 or 2, wherein the one or more organic compound(s) are selected from methane, methanol, acetic acid, butyric acid, valeric acid or combinations thereof.

4. The method according to any of the preceding claims, wherein the fermentation medium comprises volatile fatty acids, such as acetic acid, propionic acid, butyric acid and/or valeric acid.

5. The method according to any of the preceding claims, wherein the fermentation medium comprises a fluid obtained from decomposition of a biomass.

6. The method according to any of the preceding claims, wherein the fluid obtained from decomposition of a biomass is produced by hydrothermal carbonization, slow pyrolysis, hydrothermal liquefaction, biological treatment, or combinations thereof.

7. The method according to any of the preceding claims, wherein the at least one polyhydroxyalkanoate is formed from the polymerization of 3-hydroxyvalerate, 3-hydroxybutyrate, 3-hydroxyhexanoate, 3-hydroxypropionate, 3-hydroxyoctanoate or combinations thereof.

8. The method according to any of the preceding claims, wherein the at least one polyhydroxyalkanoate comprises poly(3-hydroxybutyrate-co-3-hydroxyvalerate), poly-3-hydroxyvalerate or combinations thereof.

9. The method according to any of the preceding claims comprising an extraction step after the fermentation step, said extraction step producing a fraction comprising the at least one polyhydroxyalkanoate, and a cell debris fraction.

10. The method according to claim 9, wherein the cell debris fraction obtained by the extraction is subjected to a decomposition process, such as hydrothermal carbonization, slow pyrolysis, hydrothermal liquefaction, or biological treatment, in order to produce a fluid obtained from decomposition of the cell debris fraction.

11. The method according to claim 10, wherein the cell debris fraction obtained by the extraction is mixed with a biomass, such as biosludge, before subjecting it to the decomposition process.

12. The method according to claim 10 or 11, wherein at least part of the fluid obtained from the decomposition of the cell debris fraction is used as part of the fermentation medium.

13. The method according to any of the preceding claims, wherein the converting of carbon dioxide to one or more organic compound(s) comprises or consists of microbial fermentation.

14. A product obtainable with the method according to any of the claims 1-13.

15. Use of the product according to claim 14 in or as a packaging material.
